(19) **Europäisches Patentamt / European Patent Office / Office européen des brevets**

(11) **EP 4 782 535 A1**

(12) **EUROPEAN PATENT APPLICATION**
published in accordance with Art. 153(4) EPC

(43) Date of publication:
29.07.2026 Bulletin 2026/31

(21) Application number: 24868334.4

(22) Date of filing: 20.09.2024

(51) International Patent Classification (IPC):
*C12N 5/0783* (2010.01)    *A61K 35/17* (2025.01)
*A61K 45/00* (2006.01)    *A61P 1/04* (2006.01)
*A61P 1/16* (2006.01)    *A61P 3/10* (2006.01)
*A61P 5/14* (2006.01)    *A61P 7/04* (2006.01)
*A61P 7/06* (2006.01)    *A61P 17/00* (2006.01)
*A61P 17/06* (2006.01)    *A61P 19/02* (2006.01)
*A61P 21/00* (2006.01)    *A61P 29/00* (2006.01)
*A61P 37/06* (2006.01)    *C12N 5/10* (2006.01)
*C12N 15/113* (2010.01)    *C12N 15/12* (2006.01)
*C12Q 1/02* (2006.01)

(52) Cooperative Patent Classification (CPC):
A61K 35/17; A61K 45/00; A61P 1/04; A61P 1/16;
A61P 3/10; A61P 5/14; A61P 7/04; A61P 7/06;
A61P 17/00; A61P 17/06; A61P 19/02; A61P 21/00;
A61P 29/00; A61P 37/06; C07K 14/435;    (Cont.)

(86) International application number:
PCT/JP2024/033571

(87) International publication number:
WO 2025/063257 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 22.09.2023 JP 2023158233

(71) Applicants:
• The University of Osaka
Osaka 565-0871 (JP)
• CHUGAI SEIYAKU KABUSHIKI KAISHA
Tokyo 115-8543 (JP)

(72) Inventors:
• SAKAGUCHI, Shimon
Suita-shi, Osaka 565-0871 (JP)
• CHEN, Kelvin Yigene
Suita-shi, Osaka 565-0871 (JP)
• HATA, Mayu
Suita-shi, Osaka 565-0871 (JP)
• KIBAYASHI, Tatsuya
Yokohama-shi, Kanagawa 244-8602 (JP)

(74) Representative: Hoffmann Eitle
Patent- und Rechtsanwälte PartmbB
Arabellastraße 30
81925 München (DE)

Remarks:
The complete document including Reference
Table(s) and the Sequence Listing(s) can be
downloaded from the EPO website

(54) **METHOD FOR PRODUCING REGULATORY T CELLS**

(57) The present disclosure provides a method for producing regulatory T (Treg) cells, comprising the step of suppressing the activity of RBPJ in conventional T (Tconv) cells, as well as Treg cells produced by the method. The present disclosure also provides an agent for inducing Treg cells that can be used in the method of the present disclosure. The present disclosure further provides an agent for immunosuppression comprising the Treg cells produced by the method of the present disclosure or the agent for inducing Treg cells. The agent for immunosuppression of the present disclosure is useful for treating autoimmune diseases and for treating subjects undergoing tissue transplantation.

EP 4 782 535 A1

(52)  Cooperative Patent Classification (CPC): (Cont.)
      **C12N 5/06; C12N 5/10; C12N 15/113; C12Q 1/02**

**Description**

TECHNICAL FIELD

The present application relates to a method for producing regulatory T (Treg) cells.

BACKGROUND ART

An important feature of CD25-positive CD4-positive regulatory T cells that are naturally present in the immune system is that they specifically express the transcription factor FoxP3. Deficiency or mutation of FoxP3 may impair the development and differentiation of regulatory T cells and/or the suppressive function of regulatory T cells. Experimental results showing that ectopic expression of FoxP3 in normal T cells confers immunosuppressive activity suggest that the FoxP3 gene is a master gene for the development and function of regulatory T cells. It has also been reported that, in humans, mutation of the FoxP3 gene inhibits the generation of regulatory T cells in most cases, resulting in abnormal regulation of immune responses to self antigens and non-self antigens.
It has also been reported in recent years that CD8-positive cells expressing FoxP3 play a major role in controlling immune responses.
By stimulating mouse CD4-positive T cells with an antigen in the presence of TGFβ, FoxP3 expression may be induced and induced regulatory T cells that are similar in function and phenotype to endogenous regulatory T cells may be experimentally generated. However, FoxP3 expression in the induced regulatory T cells is unstable and they cannot stably maintain immunosuppressive activity, making it difficult to apply induced regulatory T cells clinically.
Recent studies have led to the understanding that the instability of FoxP3 expression in induced regulatory T cells is mainly attributable to differences in epigenetic control of the FoxP3 gene. Epigenetics is defined as changes in gene expression or phenotype that are inherited after cell division without changes in the DNA base sequence. Epigenetic control of gene expression means that gene expression is controlled by modification of DNA or chromatin without changes in the DNA sequence. In recent years, it has become clear that lineage-specific regulation of gene expression depends on regulation by transcription factors and epigenetic regulation. Epigenetic regulation in regulatory T cells includes, for example, that regulatory regions controlling the expression of genes encoding various functional proteins specific to regulatory T cells, including FoxP3, are specifically demethylated in regulatory T cells. Based on existing knowledge, induced regulatory T cells are considered to have unstable expression of regulatory T cell-specific genes, including the FoxP3 gene, due to regulatory T cell-specific demethylated regions remaining methylated. Development of a method for generating regulatory T cells, not only with stable FoxP3 expression but also with stable function by introducing regulatory T cell-specific epigenetic modifications, is desired.
Non-Patent Literature 1 shows that FoxP3-positive cells induced *in vitro* by a mixture of IL-2, TGF-β, retinoic acid, rapamycin, and butyrate exhibit immunosuppressive activity. However, induced FoxP3 expression is unstable in that experimental system.
Non-Patent Literature 2 describes the *in vitro* production of regulatory T cells from peripheral T cells using TGF-β and rapamycin. It also proposes adoptively transferring the obtained regulatory T cells *in vivo* to suppress or treat graft-versus-host disease (GVHD) in transplantation.
Non-Patent Literature 3 reports that continued administration of rapamycin to humanized mice adoptively transferred with peripheral blood-derived T cells suppresses T-cell division via regulatory T cells and apoptosis. This literature also describes side effects of rapamycin.

PRIOR ART DOCUMENTS

Non-Patent Literature

Non-Patent Literature 1: Schmidt A, Eriksson M et al. Comparative analysis of protocols to induce human CD4+FoxP3+ regulatory T cells by combinations of IL-2, TGF-beta, retinoic acid, rapamycin and butyrate. PLoS One. 11(2): e0148474, 2016.
Non-Patent Literature 2: Hippen K L, Merkel S C et al. Generation and large-scale expansion of human induced regulatory T cells that suppress graft-versus-host disease. Am J Transplant. 11(6): 1148-57, 2011.
Non-Patent Literature 3: Hester J, Schiopu A et al. Low-dose rapamycin treatment increases the ability of human regulatory T cells to inhibit transplant arteriosclerosis in vivo. Am J Transplant. 12(8): 2008-2016, 2012.

SUMMARY OF INVENTION

Problems to be Solved by the Invention

An object of the present application is to provide a method for producing regulatory T cells. Regulatory T cells produced by the method of the present application have immunosuppressive activity and are stable.

MEANS FOR SOLVING THE PROBLEMS

The present inventors have found that stable regulatory T cells can be produced by suppressing the activity of RBPJ in conventional T cells when producing regulatory T cells from conventional T cells.

The present application provides a method for producing regulatory T cells, comprising the step of suppressing the activity of RBPJ in conventional T cells.

The present application also provides an agent for inducing regulatory T cells, comprising, as an active ingredient, a compound that suppresses the activity of RBPJ.

The present application also provides an agent for inducing regulatory T cells, comprising, as an active ingredient, conventional T cells in which the activity of RBPJ is suppressed.

The present application also provides regulatory T cells produced by the method of the present application.

The present application also provides an agent for immunosuppression, comprising, as an active ingredient, any of (a) to (c) below:

(a) regulatory T cells produced by the method of the present application;
(b) a compound that suppresses the activity of RBPJ; and
(c) conventional T cells in which the activity of RBPJ is suppressed.

The present application also provides a method for screening an agent for immunosuppression, comprising selecting a compound that suppresses the activity of RBPJ.

EFFECTS OF THE INVENTION

According to the method of the present application, it becomes possible to produce stable regulatory T (Treg) cells having immunosuppressive activity.

BRIEF DESCRIPTION OF DRAWINGS

FIG. 1 shows the percentage of FoxP3-positive cells and the FoxP3 expression level when FoxP3 expression is induced *in vitro* in PBMC-derived conventional CD4-positive T cells of a negative control and PBMC-derived conventional CD4-positive T cells in which the RBPJ gene has been knocked out.

FIG. 2 shows the percentage of FoxP3-positive cells and the FoxP3 expression level when FoxP3 expression is induced *in vitro* in PBMC-derived conventional CD4-positive T cells of a negative control and PBMC-derived conventional CD4-positive T cells in which the RBPJ gene has been knocked down.

FIG. 3 shows the demethylation rate in conserved non-coding sequence 2 (CNS2) at the FoxP3 locus when FoxP3 expression is induced *in vitro* in PBMC-derived conventional CD4-positive T cells of a negative control and PBMC-derived conventional CD4-positive T cells in which the RBPJ gene has been knocked out.

FIG. 4 shows the suppression rate of cell division of CD4-positive and CD8-positive T cells in PBMC when PBMC-derived conventional CD4-positive T cells of a negative control and PBMC-derived conventional CD4-positive T cells in which the RBPJ gene has been knocked out are co-cultured with PBMC for 3 days at the ratios described in the figure after FoxP3 expression is induced *in vitro.* The suppression rate is shown as a ratio to the negative control.

FIG. 5 shows the survival rates of NSG mice when NSG mice are transplanted with PBMC alone or PBMC plus endogenous Treg cells, a negative control, or induced Treg cells in which the RBPJ gene has been knocked out.

FIG. 6 shows the percentage of FoxP3-positive cells and the FoxP3 expression level in spleen and peripheral blood 5 days after administration to NSG mice of PBMC-derived conventional CD4-positive T cells of a negative control and PBMC-derived conventional CD4-positive T cells in which the RBPJ gene has been knocked out after FoxP3 expression is induced *in vitro.*

FIG. 7 shows the FoxP3 expression level when FoxP3 expression is induced *in vitro* in PBMC-derived conventional CD4-positive T cells in which each of the genes GPS2, NCOR2, and HDAC3 has been knocked out, and in a negative control (NTC).

FIG. 8 shows the percentage of FoxP3-positive cells and the FoxP3 expression level when FoxP3 expression is induced *in vitro* in PBMC-derived conventional CD4-positive T cells infected with a lentivirus that forcibly expresses any of GFP alone, wild-type RBPJ, or mutant RBPJ.

FIG. 9 shows the percentage of FoxP3-positive cells and the FoxP3 expression level when FoxP3 expression is induced *in vitro* after knockout of the HDAC3 gene in PBMC-derived conventional CD4-positive T cells infected with a lentivirus that forcibly expresses any of wild-type RBPJ or mutant RBPJ.

FIG. 10 shows the average peak signal of histone H3 acetylation in the vicinity of the FoxP3 locus when FoxP3 expression is induced *in vitro* in PBMC-derived conventional CD4-positive T cells of a negative control and PBMC-derived conventional CD4-positive T cells in which the RBPJ gene has been knocked out.

FIG. 11 shows the peak signal of histone H3 acetylation at and around the FoxP3 locus in the FoxP3-positive cell fraction when FoxP3 expression is induced *in vitro* in PBMC-derived conventional CD4-positive T cells infected with a lentivirus that forcibly expresses GFP alone or overexpresses the RBPJ gene.

DESCRIPTION OF EMBODIMENTS

Method for producing Treg cells

The present application provides a method for producing regulatory T (Treg) cells, comprising the step of suppressing the activity of RBPJ in conventional T (Tconv) cells.

As used herein, "conventional T (Tconv) cells" means T cells other than Treg cells, including, for example, CD4$^+$ cells and CD8$^+$ cells. Cells to be subjected to suppression of RBPJ activity include, for example, CD4$^+$ cells, CD8$^+$ cells, CD4$^+$CD25$^-$ cells, CD4$^+$CD25$^-$CD45RA$^+$ cells (naive Th cells), and CD4$^+$CD25$^-$/lowCD45RA$^-$ cells (effector Th cells). Tconv cells may be FoxP3-negative. Tconv cells are, for example, CD4$^+$ cells, CD8$^+$ cells, CD4$^+$CD25$^-$ cells, or CD4$^+$CD25$^-$CD45RA$^+$ cells.

The animal from which the Tconv cells are derived is not limited and may be, for example, mammals such as mice, rats, hamsters, guinea pigs, cattle, horses, pigs, sheep, monkeys, orangutans, chimpanzees, dogs, cats, and humans. The animals may preferably be primates, more preferably humans. In one embodiment, the Tconv cells are human Tconv cells and the Treg cells are human Treg cells.

As used herein, "regulatory T (Treg) cells" means cells that are at least positive for CD4, CD25, and FoxP3. Treg cells are, for example, induced Treg (iTreg) cells. iTreg cells are, for example, Treg cells induced to differentiate from Tconv cells in the presence of TGF-β.

As used herein, unless otherwise specified, "CD4-positive" or "CD4$^+$" refers to CD4 single-positive cells that are CD4-positive and CD8-negative, and "CD8-positive" or "CD8$^+$" refers to CD8 single-positive cells that are CD4-negative and CD8-positive.

The step of suppressing the activity of RBPJ may be, for example, a step of suppressing expression of the RBPJ gene, or a step of suppressing the function of the RBPJ protein.

In one embodiment, the step of suppressing the activity of RBPJ is a step of suppressing expression of the RBPJ gene. Suppression of gene expression can be performed by methods known per se. The step of suppressing expression of the RBPJ gene is, for example, a step of knocking out the RBPJ gene or a step of knocking down the RBPJ gene.

"Knockout of a gene" means that all or part of the gene is disrupted or mutated so that it does not exert its original function. A gene may be disrupted or mutated so that one allele on the genome does not function. Alternatively, multiple alleles may be disrupted or mutated. Knockout may be performed by known methods, including, for example, introducing into a cell a DNA construct designed to cause genetic recombination with the target gene, or using genome-editing technologies such as the CRISPR-Cas system, TALEN, and ZFN to introduce an insertion, a deletion, or a substitution. In general genome-editing technologies, a targeting vector containing the gene of interest may be used, and exogenous DNA may be inserted into a specific genomic site in a cell using homology-directed repair (HDR) after site-specific DNA cleavage by a nuclease. Introduction of a gene into cells using genome-editing technology may be performed using commercially available genome-editing kits, enzymes, and vectors.

In the CRISPR system, an endonuclease such as Cas9 or Cpf1 recognizes a specific nucleotide sequence called PAM sequence and cleaves double-stranded target DNA by the action of the endonuclease. When the endonuclease is Cas9, cleavage occurs approximately 3-4 bases upstream of the PAM sequence. Examples of the endonuclease include Cas9 from S. pyogenes, S. aureus, N. meningitidis, S. thermophilus, or T. denticola, and Cpf1 from L. bacterium ND2006 or Acidaminococcus sp. BV3L6. The PAM sequence depends on the endonuclease used; for example, the PAM sequence of S. pyogenes Cas9 is NGG. The gRNA includes, at its 5' end, a sequence of approximately 20 bases upstream of the PAM sequence (a target sequence) or a sequence complementary thereto and serves to recruit the endonuclease to the target sequence. The sequence of the portion of the gRNA other than the target sequence can be appropriately determined by a person skilled in the art depending on the endonuclease used. The gRNA may include a crRNA (CRISPR RNA) containing the target sequence or a complementary sequence thereto and responsible for the sequence specificity of the gRNA, and a tracrRNA (trans-activating crRNA) that forms a duplex and contributes to complex formation with Cas9. The crRNA and tracrRNA may exist as separate molecules. When the endonuclease is Cpf1, the crRNA alone functions as the gRNA. The gRNA sequence can be determined using tools available for selecting target sequences and designing gRNAs, such as CRISPRdirect (https://crispr.dbcls.jp/).

A vector containing a nucleic acid sequence encoding the gRNA and a nucleic acid sequence encoding the endonuclease may be introduced into cells, or alternatively, a gRNA prepared outside the cell and an endonuclease protein may be introduced into cells. The nucleic acid sequence encoding the gRNA and the nucleic acid sequence encoding the endonuclease may be present on separate vectors. Methods for introducing vectors, gRNAs, and endonucleases may include lipofection, liposome methods, electroporation, nucleofection, calcium phosphate co-precipitation, DEAE-dextran methods, microinjection, and gene gun methods.

Gene knockdown may be performed by known methods, including, for example, methods using siRNA, shRNA, antisense nucleic acids, or an expression vector capable of expressing these nucleic acid molecules to knock down the gene.

siRNA is typically a double-stranded oligo-RNA consisting of an RNA strand (antisense strand) having a sequence complementary to the nucleotide sequence of the target gene mRNA or a partial sequence thereof and a complementary strand thereof (sense strand). The nucleotide sequences of these RNAs may be appropriately designed by a person skilled in the art based on sequence information of the gene to be suppressed. The antisense strand need not be perfectly complementary to the target gene as long as suppression of the target mRNA expression can be achieved.

shRNA is a single-stranded RNA in which an RNA strand (antisense strand) having a sequence complementary to the nucleotide sequence of the target gene mRNA or a partial sequence thereof and a complementary strand thereof (sense strand) are linked by a short spacer sequence. The nucleotide sequence of shRNA may be appropriately designed by a person skilled in the art based on sequence information of the gene to be suppressed. The antisense strand need not be perfectly complementary to the target gene as long as suppression of the target mRNA expression can be achieved.

An "antisense nucleic acid" means a nucleic acid comprising a nucleotide sequence capable of specifically hybridizing with a target mRNA (mature mRNA or primary transcript) under physiological conditions of cells expressing the target mRNA, and capable of inhibiting translation of the polypeptide encoded by the target mRNA in the hybridized state. The antisense nucleic acid may be DNA or RNA, or a DNA/RNA chimera.

Examples of the mRNA sequence of the human RBPJ gene include the nucleotide sequence shown in NM_015874.6. RBPJ siRNA, RBPJ shRNA, and antisense nucleic acids of the RBPJ gene may be designed and synthesized by conventional methods based on, for example, the nucleotide sequence shown in NM_015874.6. Commercial products may be used as antisense nucleic acids of the RBPJ gene; for example, CGGACTGTGAATTCTT (SEQ ID NO: 1; Qiagen) and GTTGCCATAGAACATC (SEQ ID NO: 2; Qiagen) may be used.

Nucleotide molecules constituting siRNA, shRNA, and antisense nucleic acids may contain various chemical modifications to improve stability and activity. For example, to prevent degradation by hydrolytic enzymes such as nucleases, phosphate residues may be replaced with chemically modified phosphate residues such as phosphorothioate (PS), methylphosphonate, or phosphorodithioate. Alternatively, at least a part of them may be composed of a nucleic acid analog such as peptide nucleic acid (PNA).

Known methods may be used to introduce siRNA, shRNA, miRNA, antisense nucleic acids, or expression vectors capable of expressing these nucleic acid molecules into cells. Examples of the methods may include lipofection, electroporation, microinjection, particle gun methods, and transduction using viruses or plasmids as expression vectors. The type of expression vector is not particularly limited and known expression vectors may be used. Examples include episomal vectors, artificial chromosome vectors, viral vectors, and plasmid vectors.

Expression of the RBPJ gene may be suppressed using a compound known to suppress expression of the RBPJ gene, such as a compound that inhibits transcription of the RBPJ gene and/or its transcripts.

Gene expression may be confirmed by methods well known in the art. For example, mRNA expression may be confirmed by quantitative PCR (qPCR), RT-PCR, real-time RT-PCR, microarray, or Northern blotting, and protein expression may be confirmed by flow cytometry, ELISA, immunoprecipitation, Western blotting, or mass spectrometry.

In another embodiment, the step of suppressing the activity of RBPJ is a step of suppressing the function of the RBPJ protein. Suppression of protein function may be performed by methods known per se. For example, protein function may be suppressed by inhibiting transcriptional activity of the protein. The RBPJ protein is considered to control FoxP3 expression by forming a complex with SHARP protein, NCoR1 or NCoR2 protein, GPS2 protein, and HDAC3 protein. Accordingly, the function of the RBPJ protein may be suppressed by inhibiting formation of a complex comprising RBPJ protein, SHARP protein, NCoR1 or NCoR2 protein, GPS2 protein, and HDAC3 protein. The step of suppressing the function of the RBPJ protein is, for example, a step of inhibiting transcriptional activity of the RBPJ protein, or a step of inhibiting formation of the complex comprising RBPJ protein, SHARP protein, NCoR1 or NCoR2 protein, GPS2 protein, and HDAC3 protein.

For inhibition of transcriptional activity of the RBPJ protein, for example, a compound that inhibits binding between the RBPJ protein and a target DNA may be used, but the inhibition is not limited thereto.

Inhibition of formation of the complex comprising RBPJ protein, SHARP protein, NCoR1 or NCoR2 protein, GPS2 protein, and HDAC3 protein may be performed by methods known per se. For example, the GPS2 gene, the NCoR2 gene, or the HDAC3 gene may be knocked out using genome-editing technologies such as the CRISPR-Cas system. Alternatively, those genes may be knocked down using siRNA, shRNA, miRNA, stRNA, antisense nucleic acids, or expression vectors capable of expressing these nucleic acid molecules that target the GPS2 gene, the NCoR2 gene, or the HDAC3 gene.

Examples of the mRNA sequence of the human SHARP gene include the nucleotide sequence shown in NM_015001.3. SHARP siRNA, SHARP shRNA, and antisense nucleic acids of the SHARP gene may be designed and synthesized by conventional methods based on, for example, the nucleotide sequence shown in NM_015001.3.

Examples of the mRNA sequence of the human NCoR1 gene include the nucleotide sequence shown in NM_006311.4. NCoR1 siRNA, NCoR1 shRNA, and antisense nucleic acids of the NCoR1 gene may be designed and synthesized by conventional methods based on, for example, the nucleotide sequence shown in NM_006311.4.

Examples of the mRNA sequence of the human NCoR2 gene include the nucleotide sequence shown in NM_006312.6. NCoR2 siRNA, NCoR2 shRNA, and antisense nucleic acids of the NCoR2 gene may be designed and synthesized by conventional methods based on, for example, the nucleotide sequence shown in NM_006312.6.

Examples of the mRNA sequence of the human GPS2 gene include the nucleotide sequence shown in NM_004489.5. GPS2 siRNA, GPS2 shRNA, and antisense nucleic acids of the GPS2 gene may be designed and synthesized by conventional methods based on, for example, the nucleotide sequence shown in NM _004489.5.

Examples of the mRNA sequence of the human HDAC3 gene include the nucleotide sequence shown in NM_003883.4. HDAC3 siRNA, HDAC3 shRNA, and antisense nucleic acids of the HDAC3 gene may be designed and synthesized by conventional methods based on, for example, the nucleotide sequence shown in NM_003883.4.

Compounds known to suppress expression of the GPS2 gene, the NCoR2 gene, or the HDAC3 gene may also be used, such as compounds that inhibit transcription of those genes and/or their transcripts.

The method of the present application may be carried out *in vivo* or *in vitro*. In one embodiment, the method of the present application is carried out *in vivo*. In this embodiment, the method of the present application may comprise administering, to a subject, a compound that suppresses the activity of RBPJ.

The compound that suppresses the activity of RBPJ is, for example, a compound that suppresses expression of RBPJ or a compound that suppresses the function of the RBPJ protein. Examples of compounds that suppress expression of RBPJ include, as described above, (i) a gRNA targeting the RBPJ gene and an endonuclease, or a vector comprising nucleic acid sequences encoding the same; (ii) siRNA, shRNA, miRNA, antisense nucleic acids targeting the RBPJ gene, or expression vectors capable of expressing these nucleic acid molecules; and (iii) compounds known to suppress expression of the RBPJ gene. Examples of compounds that suppress the function of the RBPJ protein include, as described above, (i) compounds that inhibit binding between RBPJ and a target DNA; (ii) compounds that inhibit formation of a complex comprising RBPJ protein, SHARP protein, NCoR1 or NCoR2 protein, GPS2 protein, and HDAC3 protein; and (iii) siRNA, shRNA, miRNA, stRNA, antisense nucleic acids targeting the GPS2 gene, the NCoR2 gene, or the HDAC3 gene, or expression vectors capable of expressing these nucleic acid molecules.

In another embodiment, the method of the present application is carried out *in vitro*. Tconv cells may be obtained by sorting a CD4$^+$ fraction or a CD8$^+$ fraction, preferably a CD4$^+$CD25$^-$ fraction or a CD8$^+$ fraction from peripheral blood mononuclear cells of an animal including a human by, for example, flow cytometry. Tconv cells having a desired antigen specificity may be selected or induced and used. Alternatively, the Tconv cells may be Tconv cells induced from pluripotent stem cells derived from an animal including a human, such as ES cells or iPS cells. Methods for inducing T cells from pluripotent stem cells are known. Examples of methods for inducing T cells having a desired antigen specificity from pluripotent stem cells may include WO 2016/010148, WO 2016/010153, WO 2016/010154, WO 2016/010155, WO 2017/159087, WO 2017/159088, WO 2017/179720. Suppression of RBPJ expression *in vitro* may use any of the methods described above.

In this embodiment, the method of the present application may further comprise a step of inducing regulatory T (Treg) cells from Tconv cells. Known methods may be used for inducing regulatory T (Treg) cells from Tconv cells. For example, Treg cells may be induced by allowing IL-2 and TGF-β to act on Tconv cells. This step may be performed after the step of suppressing the activity of RBPJ in Tconv cells. This step may also be performed in parallel with the step of suppressing the activity of RBPJ in Tconv cells.

The step of inducing Treg cells from Tconv cells is, for example, a step of allowing IL-2 and TGF-β to act on Tconv cells. For example, IL-2 and TGF-β may be added to a medium containing Tconv cells, thereby allowing IL-2 and TGF-β to act on the Tconv cells.

In the method of the present application, cell culture medium may be a basal medium for animal cell culture supplemented with necessary factors. Examples of the basal medium may include RPMI 1640 medium, Iscove's modified Eagle's Medium, Ham's F12 medium, MEM Zinc Option medium, IMEM Zinc Option medium, IMDM medium, Medium 199, Eagle's Minimum Essential Medium (EMEM), αMEM medium, Dulbecco's modified Eagle's Medium (DMEM), Fischer's medium, and mixed media thereof.

The basal medium may contain serum (e.g., fetal bovine serum (FBS)) or may be serum-free. The serum-free medium may optionally contain one or more serum substitutes such as albumin, bovine serum albumin (BSA), transferrin, apotransferrin, KnockOut Serum Replacement (KSR) (a serum substitute used for ES cell culture) (Invitrogen), N2 supplement (Invitrogen), B27 supplement (Invitrogen), fatty acids, insulin, collagen precursors, trace elements, 2-mercaptoethanol, 3'-thioglycerol, and monothioglycerol. The basal medium may also contain one or more substances such as lipids (e.g., chemically defined lipid concentrate), amino acids, L-glutamine, GlutaMAX (Invitrogen), non-essential amino acids (NEAA), vitamins (e.g., nicotinamide and ascorbic acid), growth factors, antibiotics (e.g., penicillin and streptomycin), antioxidants, pyruvate, buffers, inorganic salts, and equivalents thereof. The basal medium may be, for example, RPMI 1640 medium.

Known TGFβ includes TGFβ1, TGFβ2, and TGFβ3. TGFβ may be, for example, TGFβ1. The concentration of TGFβ is not particularly limited and may be appropriately determined by a person skilled in the art. The concentration of TGFβ in the medium is not limited and may be about 5 pg/mL to about 500 ng/mL, about 50 pg/mL to about 500 ng/mL, or about 500 pg/mL to about 50 ng/mL; for example, about 5 ng/mL.

The concentration of IL-2 is not particularly limited and may be appropriately determined by a person skilled in the art. The concentration of IL-2 in the medium is not limited and may be about 500 mIU/mL to about 50 kU/mL, about 5 IU/mL to about 50 kU/mL, or about 50 IU/mL to about 5 kU/mL; for example, about 500 IU/mL.

Ascorbic acid and/or retinoic acid may be further added to the medium. The concentration of ascorbic acid is not limited and may be about 10 mg/mL to about 1 mg/mL, about 100 ng/mL to about 1 mg/mL, or about 1 μg/mL to about 100 μg/mL; for example, about 10 μg/mL. The concentration of retinoic acid in the medium is not limited and may be about 10 pM to about 1 μM, about 100 pM to about 1 μM, or about 1 nM to about 100 nM; for example, about 10 nM.

In the method of the present application, cell culture may be performed under general animal cell culture conditions. The culture period is not particularly limited and may be 1 to 10 days or 2 to 5 days; for example, about 3 days. The culture temperature is not particularly limited and may be about 30 to 40°C, preferably about 37°C. Cell culture is preferably performed in an atmosphere of $CO_2$-containing air, and the $CO_2$ concentration is preferably about 2 to 5%.

The Tconv cells may be, for example, cultured in suspension. As used herein, "suspension culture" means culturing cells in a non-adherent state on a culture dish. Although not particularly limited, a culture dish that has not been artificially treated to improve cell adhesion (e.g., a culture dish not coated with extracellular matrix) or a culture dish that has been artificially treated to suppress adhesion (e.g., a culture dish coated with polyhydroxyethyl methacrylate (poly-HEMA) or a polymer of 2-methacryloyloxyethyl phosphorylcholine (Lipidure)) may be used for suspension culture.

The Treg cells produced by the method of the present application may exhibit any of the characteristics (i) to (v) below, compared with Treg cells produced from Tconv cells in which the activity of RBPJ is not suppressed:

(i) a higher level of FoxP3 expression;
(ii) a more stable level of FoxP3 expression;
(iii) enhanced demethylation in the conserved non-coding sequence 2 (CNS2) region of the FoxP3 gene;
(iv) stronger immunosuppressive activity of the Treg cells; and
(v) more stable immunosuppressive activity of the Treg cells.

The level of FoxP3 expression may be confirmed by, for example, confirming expression of FoxP3 mRNA by quantitative PCR (qPCR), RT-PCR, real-time RT-PCR, microarray, or Northern blotting, or confirming FoxP3 expression by flow cytometry, ELISA, immunoprecipitation, Western blotting, or mass spectrometry. The level of FoxP3 expression is, for example, confirmed by flow cytometry.

"More stable FoxP3 expression" means that FoxP3 expression in Treg cells is maintained for a longer period. In previous studies, FoxP3 expression in induced Treg cells was unstable and those cells could not stably maintain immunosuppressive activity, which could make clinical application of induced Treg cells difficult. Stability of FoxP3 expression may be confirmed by conventional methods. For example, human Treg cells may be administered to mice and, after a certain period, the percentage of FoxP3-positive human T cells in collected samples may be confirmed.

Gene demethylation may be confirmed by methods well known in the art, for example, bisulfite sequencing. Enhanced demethylation in the CNS2 region of the FoxP3 gene means that the proportion of demethylated sites in the CNS2 region of the FoxP3 gene in Treg cells produced by the method of the present application is higher than the proportion of demethylated sites in the CNS2 region of the FoxP3 gene in Treg cells produced from Tconv cells in which the activity of RBPJ is not suppressed.

Immunosuppressive activity of Treg cells may be confirmed by methods well known in the art, for example, by evaluating the ability of Treg cells to suppress proliferation of T cells. Suppression of T-cell proliferation may be confirmed by, for example, comparing the proportion of divided T cells co-cultured with Treg cells with the proportion of divided T cells cultured alone. The T-cell proliferation-suppressing activity may be calculated using, for example, the following formula:

(T-cell proliferation-suppressing activity) = 100 × {(percentage of divided T cells cultured alone) - (percentage of divided T cells co-cultured with Treg cells)} / (percentage of divided T cells cultured alone).

The culture period is not particularly limited and may be 1 to 10 days or 2 to 5 days; for example, about 3 days.

"Stable immunosuppressive activity of Treg cells" means that the immunosuppressive activity of Treg cells is maintained for a longer period. Stability of immunosuppressive activity may be confirmed by conventional methods. For example, after inducing Treg cells and allowing a certain period to elapse, the ability of the Treg cells to suppress T-cell proliferation may be measured by the method described above to evaluate stability.

Cells

The present application also provides Treg cells produced by the method of the present application. For example, the RBPJ gene is knocked out or knocked down in the Treg cells produced by the method of the present application. The Treg cells produced by the method of the present application may exhibit any of the characteristics (i) to (v) below, compared with Treg cells produced from Tconv cells in which the activity of RBPJ is not suppressed:

(i) a higher level of FoxP3 expression;
(ii) a more stable level of FoxP3 expression;
(iii) enhanced demethylation in the conserved non-coding sequence 2 (CNS2) region of the FoxP3 gene;
(iv) stronger immunosuppressive activity of the Treg cells; and
(v) more stable immunosuppressive activity of the Treg cells.

Agents

The present application also provides an agent for inducing Treg cells, comprising, as an active ingredient, a compound that suppresses the activity of RBPJ. The compound that suppresses the activity of RBPJ is, for example, a compound that suppresses expression of RBPJ or a compound that suppresses the function of the RBPJ protein. Examples of compounds that suppress expression of RBPJ and compounds that suppress the function of the RBPJ protein are as described above.

The present application also provides an agent for inducing Treg cells, comprising, as an active ingredient, Tconv cells in which the activity of RBPJ is suppressed.

The present application also provides an agent for immunosuppression, comprising, as an active ingredient, any of (a) to (c) below:

(a) Treg cells produced by the method of the present application;
(b) a compound that suppresses the activity of RBPJ; and
(c) Tconv cells in which the activity of RBPJ is suppressed.

The agent of the present application may be used for treating a subject having an autoimmune disease. The autoimmune disease may be, for example, any of Addison's disease, ankylosing spondylitis, autoimmune hepatitis, dermatitis, Goodpasture syndrome, Graves' disease, Guillain-Barré syndrome, Hashimoto's thyroiditis, hemolytic anemia, juvenile arthritis, juvenile myositis, Kawasaki disease, inflammatory bowel disease (such as Crohn's disease and ulcerative colitis), polymyositis, alveolar proteinosis, multiple sclerosis, myasthenia gravis, neuromyelitis optica, psoriasis vulgaris, psoriatic arthritis, rheumatoid arthritis, Sjögren's syndrome, systemic scleroderma, systemic sclerosis, systemic lupus erythematosus, thrombotic thrombocytopenic purpura (TTP), type I diabetes, uveitis, vasculitis, and vitiligo vulgaris. The agent of the present application may also be used for treating a subject undergoing tissue transplantation, and may be used, for example, for suppressing graft rejection and/or graft-versus-host disease (GVHD) in a subject undergoing tissue transplantation.

The agent of the present application may be frozen. As methods for freezing and thawing a compound that suppresses the activity of RBPJ, Tconv cells, and Treg cells, known methods may be used. When the agent of the present application comprises Tconv cells or Treg cells, the agent of the present application may comprise, for example, a cryoprotectant such as DMSO, glycerol, polyvinylpyrrolidone, polyethylene glycol, albumin, dextran, and sucrose.

The agent of the present application may comprise a pharmaceutically acceptable carrier or additive. Examples of these carriers or additives include, for example, isotonic agents, thickening agents, saccharides, preservatives, pH adjusters, stabilizers, gel bases, wetting agents, suspending agents, excipients, buffering agents, and antioxidants. Specific examples of carriers or additives are not particularly limited, and include water for injection, physiological saline, 5% glucose solution, Ringer's solution, lactated Ringer's solution, acetate Ringer's solution, bicarbonate Ringer's solution and amino acid solutions. The pharmaceutically acceptable carrier or additive may be used alone or as a mixture of two or more thereof.

The agent of the present application may be, for example, an injection, a liquid preparation, an oral preparation, a patch preparation, a preparation for transplantation, and a gel preparation.

The route of administration of the agent of the present application may be, for example, intravenous administration, subcutaneous administration, intramuscular administration, intraarterial administration, lymphatic administration, intrathecal administration, intraperitoneal administration, rectal administration, vaginal administration, transdermal administration, implantation, direct administration to an organ, and local transplantation.

The dose and frequency of administration of the agent of the present application may be appropriately set by a person skilled in the art according to, for example, the animal species, the health condition, age, body weight of the subject, route of administration, and dosage form, so that an effective amount of the active ingredient is administered to the subject. For example, the agent of the present application may be administered once to several times per day, or once to several times per day or once to several times per several days or once to several times per one week or several weeks, for example, once every 1 to 4 weeks, but is not limited thereto. The effective amount in a certain situation may be readily determined by routine experimentation, and is within the skill and judgment of an ordinary clinician. For example, when the agent of the present application comprises cells as an active ingredient, the effective amount is not particularly limited, but may be, for example, about $10^4$ cells/kg body weight to about $10^7$ cells/kg body weight, in terms of the number of cells.

As used herein, "treat" or "treatment" means, in a subject having a disease, reducing or eliminating the cause of the disease, delaying or stopping the progression thereof, and/or reducing, alleviating, improving, or eliminating the symptoms thereof.

Subjects to be treated for a disease may be mammals such as mice, rats, hamsters, guinea pigs, cattle, horses, pigs, sheep, monkeys, orangutans, chimpanzees, dogs, cats, and humans, preferably primates, more preferably humans.

The composition may be used alone or in combination with one or more additional active ingredients, particularly active ingredients for treatment or prevention of a disease. "In combination" with components means not only the use of a dosage form comprising all components and the use of a combination of dosage forms each comprising each component separately, but also means administration of each component simultaneously, sequentially, or with delayed administration of any component, as long as they are used for treatment and/or prevention of a disease. It is also possible to use two or more additional active ingredients in combination. Active ingredients suitable for combination include, for example, anti-inflammatory agents, antibacterial agents, antifungal agents, antiviral agents, immunosuppressive agents, and molecular targeted drugs.

(Screening method)

The present application also provides a method for screening an agent for immunosuppression, comprising selecting a compound that suppresses the activity of RBPJ. The screening method of the present application may comprise, for example, the steps of: (a) measuring the activity of RBPJ in the presence and absence of a test compound; and (b) selecting the compound when the activity of RBPJ is suppressed in the presence of the test compound compared with in the absence of the test compound. The measurement in step (a) is performed, for example, using cells expressing RBPJ.

The "test compound" may be any compound that may be a candidate for a compound that suppresses immunity, and the type thereof is not particularly limited. The test compound may be, for example, any of an organic compound, an inorganic compound, a nucleic acid (such as siRNA, shRNA, miRNA, antisense, decoy, aptamer, and ribozyme), a protein (such as an antibody), a peptide (linear peptide, cyclic peptide), a sugar chain, a lipid, or a combination thereof.

The activity of RBPJ may be measured, for example, by a reporter gene assay using an RBPJ binding sequence and the like. Specifically, the transcription start site region of the FoxP3 locus is isolated and inserted upstream of a luciferase gene to prepare a luciferase gene expression vector for a reporter gene assay. The transcription start site region of the FoxP3 locus comprises an RBPJ binding sequence. When expression of the luciferase gene may be detected after introducing the above expression vector into cells, the activity of RBPJ may be measured using this assay system.

(Kit)

The present application also provides a kit for inducing Treg cells, comprising a compound that suppresses the activity of RBPJ. The compound that suppresses the activity of RBPJ may be, for example, a compound that suppresses expression of RBPJ or a compound that suppresses the function of the RBPJ protein. Examples of compounds that suppress expression of RBPJ and compounds that suppress the function of the RBPJ protein are as described above. The kit of the present application may further comprise, for example, a buffer, a reaction vessel, and an instruction manual.

(Therapeutic method)

The present application also provides a method for treating an autoimmune disease, comprising administering, to a subject in need thereof, Treg cells produced by the method of the present application, a compound that suppresses the activity of RBPJ, and/or Tconv cells in which the activity of RBPJ is suppressed. The present application also provides use of Treg cells produced by the method of the present application, a compound that suppresses the activity of RBPJ, and/or Tconv cells in which the activity of RBPJ is suppressed, for producing an agent for treating an autoimmune disease. The present application also provides Treg cells produced by the method of the present application, a compound that suppresses the activity of RBPJ, and/or Tconv cells in which the activity of RBPJ is suppressed, for use in treating an autoimmune disease. Examples of autoimmune diseases and compounds that suppress the activity of RBPJ are as described above.

(Preventive method)

The present application also provides a method for preventing an autoimmune disease, the method comprising administering, to a subject in need thereof, Treg cells produced by the method of the present application, a compound that suppresses the activity of RBPJ, and/or Tconv cells in which the activity of RBPJ is suppressed. The present application also provides use of Treg cells produced by the method of the present application, a compound that suppresses the activity of RBPJ, and/or Tconv cells in which the activity of RBPJ is suppressed, for producing an agent for preventing an autoimmune disease. The present application also provides Treg cells produced by the method of the present application, a compound that suppresses the activity of RBPJ, and/or Tconv cells in which the activity of RBPJ is suppressed, for use in preventing an autoimmune disease. Examples of autoimmune diseases and compounds that suppress the activity of RBPJ are as described above.

Examples are described below to further illustrate the present invention in more detail; however, the present invention is not limited in any way by the Examples.

Example 1: Enhanced FoxP3 expression by RBPJ gene knockout

Human peripheral blood mononuclear cells (PBMCs, Cellular Technology Ltd.) were thawed and concentrated using the CD4$^+$ T Cell Isolation Kit (Miltenyi Biotec) to isolate CD4$^+$ T cells. The processed cells were stained at 4°C for 30 minutes using anti-CD4 antibody (RPA-T4, Biolegend), anti-CD25 antibody (M-A251, BD Pharmigen), anti-CD45RA antibody (HI100, Biolegend), and anti-CD127 antibody (HIL-7R-M21, BD Bioscience). CD4$^+$ CD25$^-$ CD45RA$^+$ naïve Tconv cells were isolated by flow cytometry using the FACS Aria Fusion (BD). The isolated naive Tconv cells were suspended in RPMI 1640 (Nacalai Tesque) comprising 10% FBS (Cytiva)/ 2 mM GlutaMAX/ 1 mM sodium pyruvate/ 0.1 mM nonessential amino acids/ 55 μM β-mercaptoethanol/ 100 U/ml penicillin/ 100 μg/ml streptomycin/10 mM HEPES (Gibco) (hereinafter referred to as T cell medium) supplemented with 500IU/ml Interleukin-2 (IL-2, R&D systems) to achieve a cell density of $1\times10^6$ cells/mL. The cells were then mixed with Dynabeads Human T-Activator CD3/CD28 (hereinafter referred to as CD3/CD28 Dynabeads, Thermo Fisher Scientific) at a 1:1 ratio and cultured for 3 days at 37°C in a 5% $CO_2$ incubator. After the initial incubation, the CD3/CD28 Dynabeads were removed, and the cells were cultured for an additional 6-8 days under the same conditions. During the culture period, an equal volume of fresh T cell medium supplemented with 1000 IU/mL IL-2 was added daily.

The ribonucleoprotein (RNP) for RBPJ gene knockout was prepared by mixing 400 μM crRNA (GAAGAATT CACAGTCCGAGA (SEQ ID NO: 3), AGATACTTGCATGTAGAAGG (SEQ ID NO: 4), IDT) and 400 μM tracrRNA (IDT) at 1:1 (v/v), incubating the mixture at 95°C for 5 minutes, and returning the mixture to room temperature, and then mixing 2.7 μL of the resulting 200 μM gRNA complex with 30 μg of Cas9 protein (Takara Bio Inc.), followed by incubation at room temperature for 15 minutes or longer. As a negative control, Alt-R CRISPR-Cas9 Negative Control crRNA #1 and #2 (both IDT) were used as crRNAs. Unless otherwise specified, two RNPs for the same target were mixed in equal amounts and used such that the amount of Cas9 used was 30 μg.

The cultured naïve Tconv cells were suspended in P2 Primary Cell Nucleofector Solution (Lonza) so as to obtain a concentration of 1-$2\times10^6$ cells/20 μL, and mixed with the prepared RNP. The mixture was transferred to a 16-well Nucleocuvette strip, and the RNP was introduced into the cells by electroporation using a 4D Nucleofector (program EH-100, Lonza). The treated cell suspension was suspended in T cell medium so as to obtain a concentration of $1\times10^6$ cells/mL, and cultured at 37°C for 6-8 days in a 5% $CO_2$ incubator. During the culture period, half of the medium was replaced daily with fresh T cell medium supplemented with 1000 IU/mL IL-2.

After culture, dead cells were removed from the cultured cells using an EasySep Dead Cell Removal (Annexin V) Kit (STEMCELL Technologies), and the cells were then suspended in T cell medium containing Dynabeads CD3/CD28 (at a ratio of 1:1), 500 IU/mL IL-2, 5 ng/mL transforming growth factor-β (hereinafter referred to as "TGF-β", R&D Systems), 10 μg/mL ascorbic acid (Sigma-Aldrich), 10 nM all-trans retinoic acid (hereinafter referred to as "ATRA", Sigma-Aldrich), 5 μg/mL anti-interferon-γ antibody, anti-interleukin-4 antibody, anti-interleukin-6 antibody, anti-tumor necrosis factor-α antibody, and anti-Fas ligand antibody (all BioLegend), so as to obtain a concentration of $1\times10^6$ cells/mL, and cultured at 37°C for 3 days in a 5% $CO_2$ incubator to induce FoxP3 expression.

The cells after FoxP3 induction were stained using the Zombie-NIR Fixable Viability Kit (BioLegend) and anti-FoxP3 antibody (236A/E7, eBioscience) respectively at 4°C for 30 minutes. The staining was conducted by using the Foxp3/Transcription Factor Staining Buffer Set (eBioscience) according to the package insert. Stained cells were analyzed by flow cytometry using FACS Celesta (BD).

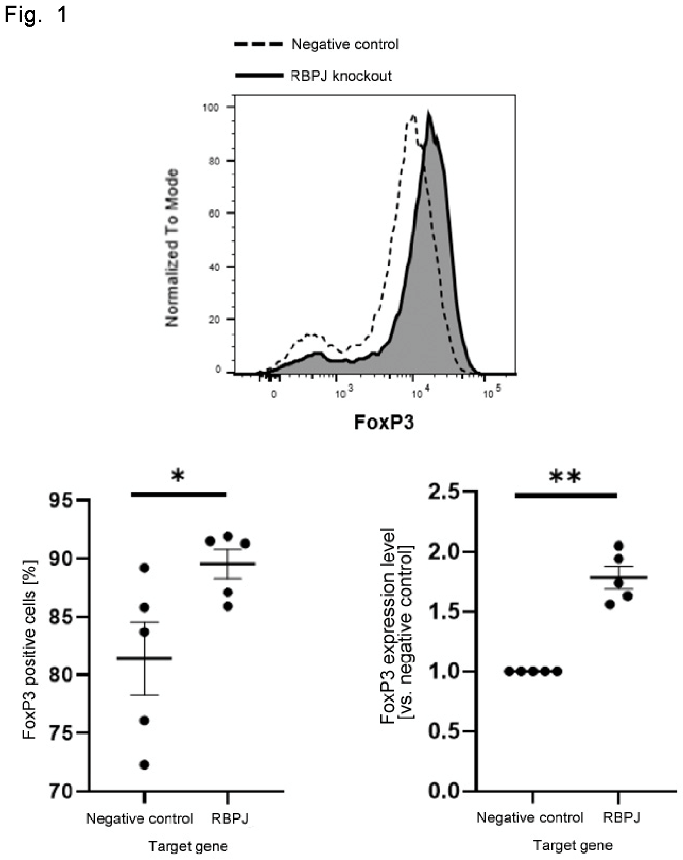

From the results, it was confirmed that knockout of the RBPJ gene resulted in a higher FoxP3-positive rate and higher FoxP3 expression (Fig. 1). This means that induced Treg cells may be obtained more efficiently by knockout of the RBPJ gene. In addition, since the expression level of FoxP3, which is a central transcription factor of Treg cells, is high, it is suggested that the cells may be functionally close to endogenous Treg cells.

Example 2. Enhanced FoxP3 expression by RBPJ gene knockdown

Naive Tconv cells prepared in the same manner as in Example 1 were subjected to FoxP3 expression induction on the day of preparation in the same manner as in Example 1. Knockdown of the RBPJ gene was carried out by adding 2 μM antisense oligonucleotides (CGGACTGTGAATTCTT (SEQ ID NO: 1), GTTGCCATAGAACATC (SEQ ID NO: 2), Qiagen) in addition to the FoxP3 expression induction stimulation. As a negative control, Negative control A Antisense LNA GapmeR (Qiagen) was used. The cells after FoxP3 induction were analyzed by flow cytometry in the same manner as in Example 1.

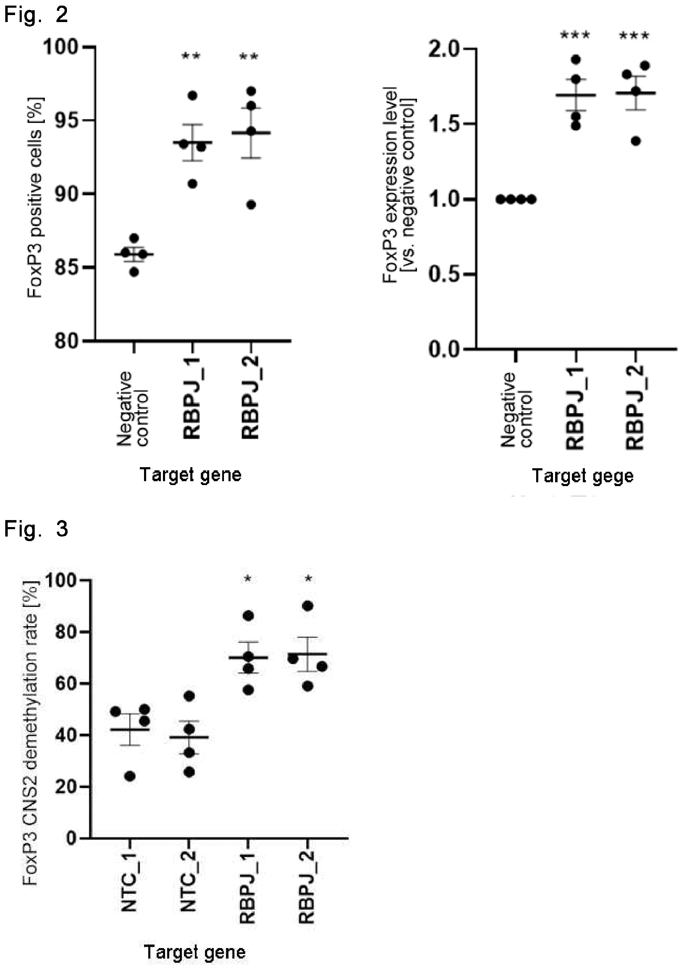

From the results, it was confirmed that knockdown of the RBPJ gene resulted in a higher FoxP3-positive rate and higher FoxP3 expression (Fig. 2). This means that induced Treg cells may be obtained more efficiently by knockdown of the RBPJ gene. In addition, since the expression level of FoxP3, which is a central transcription factor of Treg cells, is high, it is suggested that the cells may be functionally close to endogenous Treg cells.

Example 3. Enhanced demethylation of FoxP3 conserved non-coding sequence 2 (CNS2) by RBPJ gene knockout

FoxP3 induction in naïve Tconv cells in which the RBPJ gene had been knocked out was carried out in the same manner as in Example 1. After 3 days of FoxP3 induction stimulation, CD3/CD28 Dynabeads were removed, and the cells were cultured at 37°C for 7 days in a 5% $CO_2$ incubator in fresh T cell medium supplemented with 500 IU/mL IL-2 and 10 μg/mL ascorbic acid. During the culture period, half of the medium was replaced daily with fresh T cell medium supplemented with 1000 IU/mL IL-2 and 20 μg/mL ascorbic acid. After culture, the cells were stained in the same manner as in Example 1, and a live FoxP3-positive fraction was sorted using FACS Aria Fusion. The sorted cells were decrosslinked for 20 hours or longer, and DNA was extracted using NucleoSpin Tissue XS (Takara Bio Inc.). The extracted DNA was subjected to bisulfite conversion using EZ DNA Methylation-Lightning Kit (Zymo Research), and then the FoxP3 CNS2 region was amplified by PCR reaction (Fw Primer: 5'-TTGGGTTAAGTTTGTTGTAGGATAG-3' (SEQ ID NO: 5), Rv Primer: 5'-ATCTAAACCCTATTATCACAACCCC-3' (SEQ ID NO: 6), FASMAC). The amplified amplicon was excised from an agarose gel after agarose gel electrophoresis, and purified using QIAquick Gel Extraction Kit (Qiagen). The purified amplicon was subjected to a TA cloning reaction (16°C, 1 hour) using pTAKN-2 DynaExpress TA Cloning Kit (Bio Dynamics Laboratory Inc.), and the ligation mixture was transformed into Escherichia coli (DH5α, Takara Bio Inc.) (42°C, 45 seconds). The E. coli were seeded on LB plates containing kanamycin and cultured at 37°C for 17-20 hours. After culture, 16-24 single colonies per sample were selected, and a sequencing template was prepared using illustra TempliPhi 100 Amplification Kit (Cytiva). The prepared template was subjected to sequencing treatment using BigDye Terminator v3.1 Cycle Sequencing Kit (Applied Biosystems). The treated samples were subjected to sequence analysis by Sanger sequencing using Applied Biosystems 3500 (Applied Biosystems). The obtained sequence data were used to confirm the presence or absence of demethylation using, as an indicator, whether base conversion by bisulfite treatment had occurred at 11 CpG methylation sites present in the CNS2 region, and the demethylation rate was calculated from data of 12 clones per sample.

From the results, it was shown that demethylation of the CNS2 region of the FoxP3 gene locus was promoted in induced Treg cells in which the RBPJ gene had been knocked out (Fig. 3). Since it has been reported that demethylation of CNS2 is involved in the stability of FoxP3 expression (Ohkura et al., Immunity. 2012 Nov 16;37(5):785-99.), it is presumed that more stable induced Treg cells were obtained.

Example 4. Enhanced T-cell proliferation suppressive activity in RBPJ gene knockout induced Treg cells

Naïve Tconv cells prepared in the same manner as in Example 3 were subjected to FoxP3 induction, followed by culture for 7 days. The induced Treg cells after culture were fluorescently labeled by incubation at 37°C for 20 minutes in CellTrace Far Red Cell Proliferation Kit (Thermo Fisher Scientific) diluted 1000-fold with PBS. Human-derived PBMC were thawed and fluorescently labeled in the same manner using CellTrace Violet Cell Proliferation Kit (Thermo Fisher Scientific). First, 100 μL of PRIME-XV T Cell Expansion XSFM (serum-free T cell medium, Irvine Scientific) containing 20 IU/mL IL-2 and 1 μg/mL anti-CD3 antibody (OKT3, BioLegend) was added to each well of a V-bottom 96-well plate. The fluorescently labeled PBMC were suspended in serum-free T cell medium so as to obtain a concentration of $1 \times 10^5$ cells/50

µL, and seeded into the V-bottom 96-well plate. The fluorescently labeled induced Treg cells were suspended in 50 µL of serum-free T cell medium in the same manner such that PBMC:induced Treg cells became 1:0.5 to 1:0.0625 (common ratio 2), and seeded into the above plate. The cell suspension in each well was again thoroughly suspended by pipetting, and cultured at 37°C for 3 days in a 5% $CO_2$ incubator. After culture, the cells were stained at 4°C for 30 minutes using Zombie-Green Fixable Viability Kit (BioLegend), an anti-CD4 antibody (RPA-T4), and an anti-CD8 antibody (RPA-T8), and analyzed by flow cytometry using FACS Celesta.

For each sample, the ratio of cells that had divided one or more times (percentage proliferation, % Proliferation) was calculated for CD4-positive T cells and CD8-positive T cells that were negative for CellTrace Far Red, and the suppression rate (% Suppression) was calculated by calculating 100×{(average value of % Proliferation in wells without addition of induced Treg cells) - (% Proliferation in the target well) }/(average value of % Proliferation in wells without addition of induced Treg cells). Furthermore, the value of each well (Ratio of % Suppression) was calculated by setting the suppression rate of the well to which negative control induced Treg cells were added at PBMC:induced Treg cells = 1:0.5 as 1, and the graph was plotted.

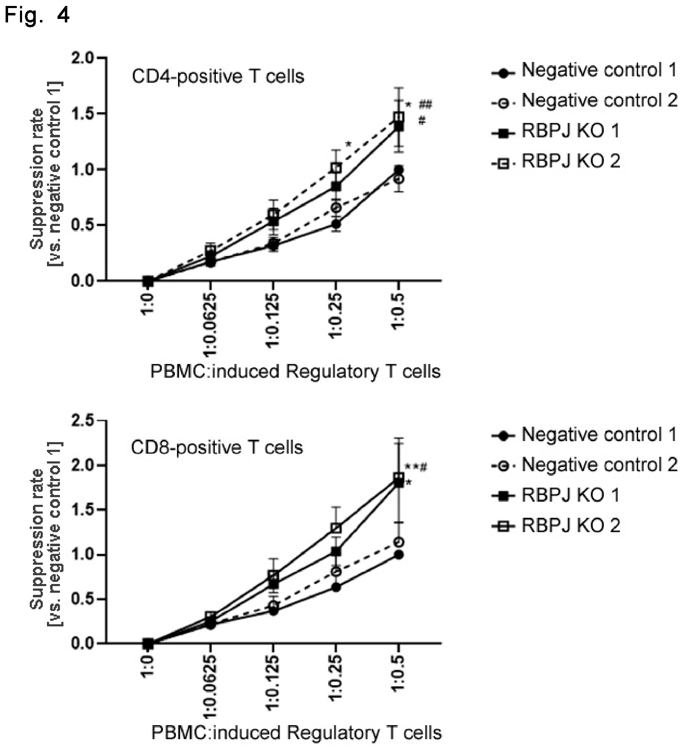

From the results, it was confirmed that induced Treg cells in which the RBPJ gene had been knocked out had a higher activity to suppress T-cell proliferation as compared with the negative control (Fig. 4).

Example 5. Effect of RBPJ gene knockout induced Treg cells on graft-versus-host disease (GVHD)

Graft-versus-host disease (GVHD) was induced by administering human-derived PBMC to NOD.Cg-Prkdcscid-dIl2rgtm1 WjI/SzJ mice (NSG mice, 7 weeks old, male, Jackson Laboratory Japan). Human-derived PBMC were thawed, and cell clumps generated by freeze-thawing were removed by passing through a 40 µm cell strainer. Induced Treg cells were prepared in the same manner as in Example 3. Endogenous Treg cells were stained in the same manner as in Example 1, and then sorted by flow cytometry using FACS Aria Fusion as a fraction of CD4-positive CD127-negative and low-expression cells, excluding CD25 intermediate-expression CD45RA-negative cells. Endogenous Treg cells were suspended in T cell medium containing 500 IU/mL IL-2 so as to obtain a cell density of $1 \times 10^6$ cells/mL, mixed with Dynabeads Human Treg Expander (hereinafter referred to as "Treg expander beads", Thermo Fisher Scientific) at a ratio of 1:1, and cultured at 37°C for 8 days in a 5% $CO_2$ incubator. After 8 days, the Treg expander beads were removed, and the cells were further cultured for 8-10 days under the same conditions. During the culture period, half of the medium was replaced or an equal volume was added daily with fresh T cell medium supplemented with 1000 IU/mL IL-2. Dead cells were removed from induced or endogenous Treg cells using an EasySep Dead Cell Removal (Annexin V) Kit. PBMC, induced Treg cells, and endogenous Treg cells were each suspended in PBS so as to obtain a concentration of $2.5 \times 10^7$ cells/mL, and a cell mixture of 200 µL per mouse was prepared by mixing 100 µL each of PBMC and Treg cells. One day before cell administration, NSG mice were irradiated with X-rays at 2.25 Gy using MBR-1520R-3 (Hitachi Power Solutions). On the day after X-ray irradiation, NSG mice were intravenously administered with 200 µL of a mixture of $2.5 \times 10^6$ PBMC and $2.5 \times 10^6$ induced or endogenous Treg cells. After administration of the cell mixture, the body weight and survival rate of the mice were measured at arbitrary intervals for 30 days. When body weight decreased by 20% or more, euthanasia was performed and the mouse was treated as a dead individual.

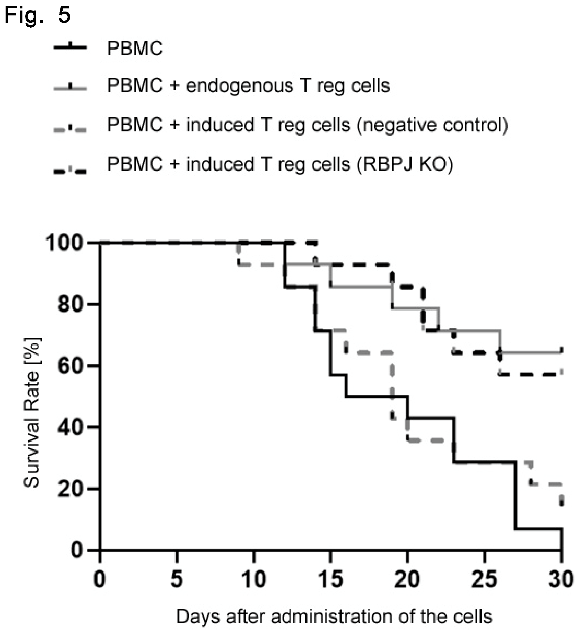

As a result, negative control induced Treg cells did not show an effect on reduction in survival rate due to GVHD; however, it was confirmed that induced Treg cells in which the RBPJ gene had been knocked out improved survival rate at a level equivalent to that of endogenous Treg cells (Fig. 5).

Example 6. Stability of RBPJ gene knockout induced Treg cells in vivo

The stability in vivo of induced Treg cells in which the RBPJ gene had been knocked out was verified by intravenously administering induced Treg cells to NSG mice. The induced Treg cells to be administered were prepared in the same manner as in Example 5, and administration to mice was carried out by administering 100 µL of the cell suspension per mouse. Five days after cell administration, spleens were collected from the mice and blood was collected from the inferior vena cava. The spleen was crushed on a 70 µm mesh to prepare a cell suspension, and the suspension was suspended in 500 µL of red blood cell lysis buffer Hybri-Max (Sigma-Aldrich) and incubated at room temperature for 5 minutes to perform hemolysis treatment. The cells after hemolysis treatment were washed with PBS containing 2% bovine serum albumin and then used for staining. The collected 500-700 µL of peripheral blood was added to 10 mL of ACK Lysing Buffer (Gibco) and incubated at room temperature for 10 minutes to perform hemolysis treatment. Similarly, the cells after hemolysis treatment were washed with PBS containing 2% bovine serum albumin and then used for staining. Cells derived from spleen and peripheral blood were mixed, stained in the same manner as in Example 1 using Zombie-Green Fixable Viability Kit, anti-CD4 antibody (RPA-T4), anti-FoxP3 antibody (236A/E7), and anti-mouse CD45 antibody (30-F11), and analyzed by flow cytometry using FACS Celesta.

The administered induced Treg cells were analyzed as a human CD4-positive mouse CD45-negative fraction, and the FoxP3-positive rate in the corresponding fraction was calculated.

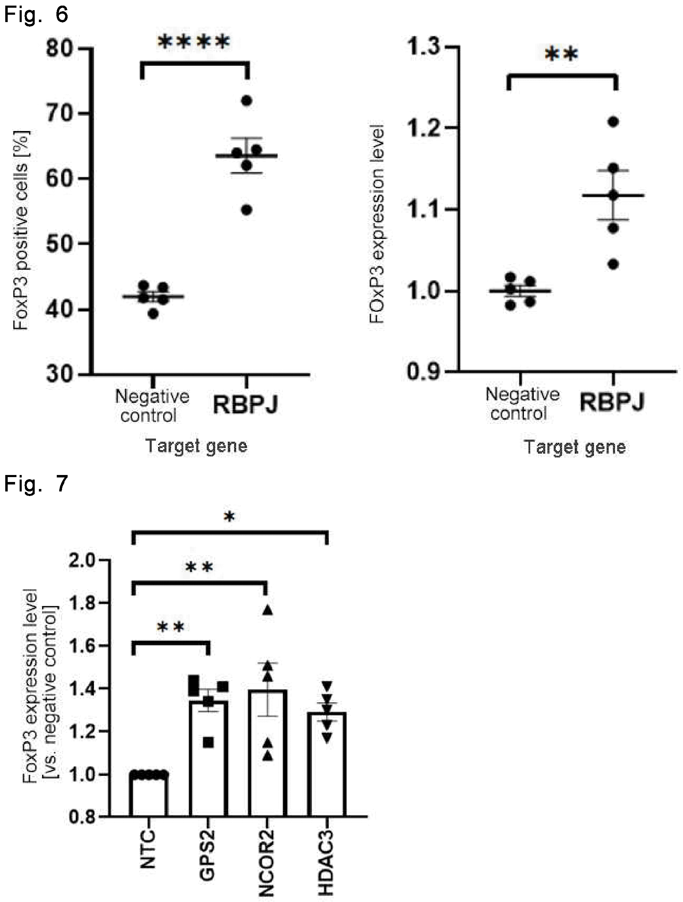

From the results, as inferred from the enhancement of CNS2 demethylation at the FoxP3 gene locus in Example 2, it was shown that induced Treg cells in which the RBPJ gene had been knocked out stably expressed FoxP3 in vivo (Fig. 6). This is considered to be involved in retention of the suppressive activity of induced Treg cells, and is consistent with the effect on GVHD shown in Example 5.

Example 7. Enhanced FoxP3 expression by knockout of GPS2, NCOR2, and HDAC3 genes

In the same manner as in Example 1, knockout was carried out for each of the GPS2 gene (gRNA1: GCTG CACCGGCACATTATGATGG (SEQ ID NO: 7), gRNA2: AACGGAGGCGAAAGGAACAGAGG (SEQ ID NO: 8)), the NCOR2 gene (gRNA1: GGAACACGGCCGCAACTGGTCGG (SEQ ID NO: 9), gRNA2: AGAAGGCTGAAGCTGCAC ATCGG (SEQ ID NO: 10)), and the HDAC3 gene (gRNA1: GTAATGCAGGACCAGGCTATGGG (SEQ ID NO: 11), gRNA2: GACGTGGGCAACTTCCACTACGG (SEQ ID NO: 12)) in PBMC-derived conventional CD4-positive T cells, and then induction of FoxP3 expression was carried out and expression analysis was performed.

As a result, it was confirmed that higher FoxP3 expression was induced in cells in which any of the genes GPS2, NCOR2, and HDAC3, which are constituent components of the SMRT-NCoR repressive complex, had been knocked out (Fig. 7).

Example 8. Involvement of SMRT-NCoR repressive complex in suppression of FoxP3 expression by RBPJ

Naïve Tconv cells prepared in the same manner as in Example 1 were stimulated for 24 hours at 37°C in a 5% $CO_2$ incubator using an anti-CD3 antibody (10 $\mu$g/mL, UCHT1, BioLegend) and an anti-CD28 antibody (5 $\mu$g/mL, CD28.2, BioLegend) plate-coated at 37°C for 2 hours. Thereafter, lentiviruses expressing green fluorescent protein (GFP), wild-type RBPJ, or mutant RBPJ having F235A/L362A amino acid substitution mutations were infected by centrifugation infection (33°C, 1400 g, 2 hours). Since the wild-type and mutant RBPJ-expressing viruses simultaneously transcribe a 2A self-cleaving sequence and the GFP gene, GFP may be utilized as a reporter. Two days after the infection operation, the cells were cultured at 37°C for 6-8 days in a 5% $CO_2$ incubator in fresh T cell medium containing 500 IU/mL IL-2. After culture, a live GFP-positive fraction was sorted by flow cytometry using FACS Aria Fusion. The sorted GFP-positive Tconv cells were subjected to FoxP3 expression induction in the same manner as in Example 1, and FoxP3 expression analysis was performed using FACS Celesta. The amino acid sequences of wild-type RBPJ and mutant RBPJ are shown in SEQ ID NOs: 13 and 14, respectively.

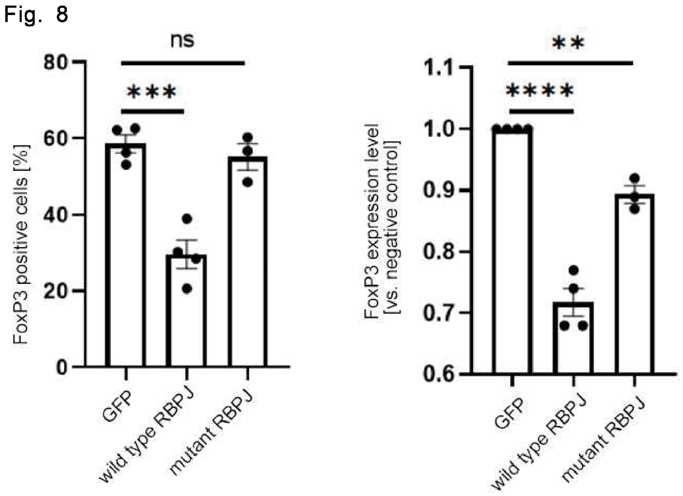

As a result, FoxP3 induction was markedly reduced in Tconv cells expressing wild-type RBPJ, whereas almost no effect was observed when mutant RBPJ was expressed (Fig. 8). Since it has been reported that F235/L362 is important for formation of the SMRT-NCoR repressive complex via SHARP (Yuan et al., Cell Rep. 2019 Jan 22;26(4):845-854.e6.), it was confirmed that the SMRT-NCoR repressive complex is involved in suppression of FoxP3 expression by RBPJ.

Example 9. Effect of HDAC3 gene knockout on suppression of FoxP3 expression by RBPJ overexpression

In the same manner as in Example 8, naïve Tconv cells were infected with lentiviruses expressing wild-type or mutant RBPJ. After culture for 8-10 days, RNP containing HDAC3 gRNA (gRNA1: GTAATGCAGGACCAGGCTAT (SEQ ID NO: 15), gRNA2: GACGTGGGCAACTTCCACTA (SEQ ID NO: 16)) was introduced into the cells by electroporation in the same manner as in Example 1. After culture for 6-8 days, a live GFP-positive fraction was sorted by flow cytometry using FACS Aria Fusion. The sorted GFP-positive Tconv cells were subjected to FoxP3 expression induction in the same manner as in Example 1, and FoxP3 expression analysis was performed using FACS Celesta.

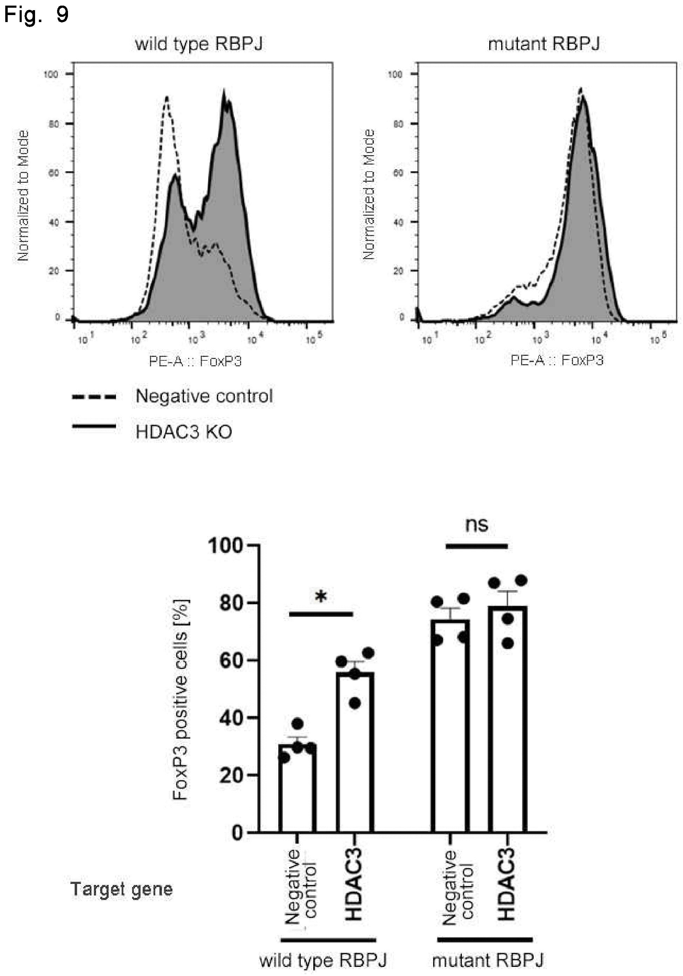

As a result, whereas no effect of HDAC3 gene knockout was observed during mutant RBPJ overexpression, it was confirmed that the decrease in FoxP3-positive rate observed during wild-type RBPJ overexpression was partially restored by HDAC3 knockout (Fig. 9). Since HDAC3 is one of the constituent components of the SMRT-NCoR repressive complex, it was shown that FoxP3 expression may be controlled by targeting the SMRT-NCoR repressive complex including RBPJ in suppression of FoxP3 expression by RBPJ.

Example 10. Change in histone acetylation at FoxP3 gene locus by RBPJ gene knockout Induced Treg cells in which the RBPJ gene had been knocked out were prepared in the same manner as in Example 1. The prepared cells were fixed by incubation at room temperature for 10 minutes in 1% formaldehyde. After fixation, the cells were washed with PBS containing 2% bovine serum albumin, suspended in 100 $\mu$L of nuclear lysis buffer (50 mM Tris-HCl pH 7.5, 10 mM EDTA, 1% SDS) containing cOmplete protease inhibitor cocktail (Roche), and incubated on ice for 10 minutes. The cells were sonicated using a Picoruptor (Diagenode) by repeating 5 cycles of sonication for 30 seconds and incubation for 30 seconds. After sonication, the cells were centrifuged at 4°C and 10000 g for 10 minutes, and the supernatant was collected to prepare a solution containing sheared chromatin. Chromatin immunoprecipitation was carried out using iDeal ChIP-seq kit for Histones (Diagenode) according to the attached instructions. An anti-histone H3 acetylation antibody (Millipore) was used as an antibody for immunoprecipitation. A sequencing library was prepared from the immunoprecipitated DNA solution obtained above using KAPA HyperPrep kit (NIPPON Genetics) according to the attached instructions. The prepared library was subjected to sequence analysis using NextSeq500 (Illumina) or NovaSeq (Illumina). From the obtained sequence data, peak signals of histone H3 acetylation on the human genome were calculated.

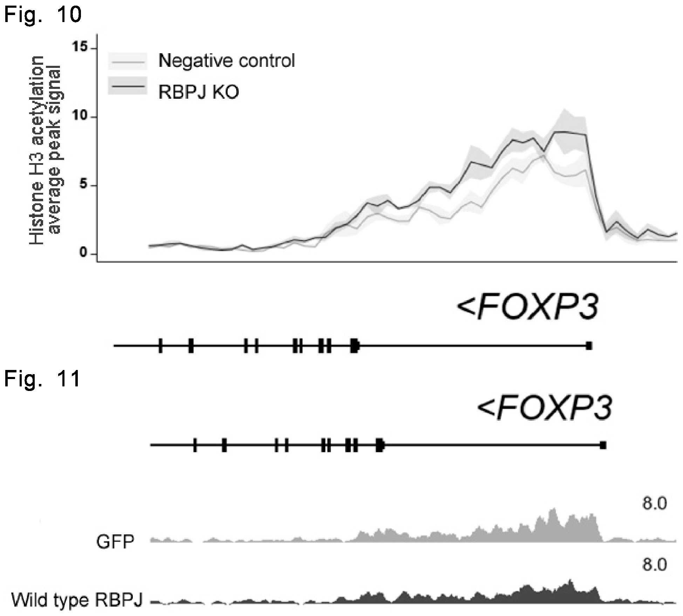

As a result, it was confirmed that, in induced Treg cells in which the RBPJ gene had been knocked out, acetylation of histone H3 was enhanced at the FoxP3 gene locus and in the vicinity thereof as compared with the negative control (Fig. 10). In general, it is considered that changes in histone structure caused by histone acetylation promote transcription by enabling access of transcription factors and other transcription activators to the genome, and this is regulated by HDAC molecules (Nat Rev Mol Cell Biol. 2015 Apr;16(4):258-64.). Therefore, this, together with Examples 8-10, indicates that FoxP3 expression in induced Treg cells is controlled by histone H3 acetylation at the FoxP3 gene locus and in the vicinity thereof by the SMRT-NCoR repressive complex including RBPJ and HDAC3.

Example 11. Change in histone acetylation at FoxP3 gene locus by RBPJ overexpression Induced Treg cells in which the RBPJ gene was overexpressed were prepared in the same manner as in Example 6. The prepared cells were fixed by incubation at room temperature for 10 minutes in 1% formaldehyde. After fixation, the cells were washed with PBS containing 2% bovine serum albumin, suspended in lysis/permeabilization buffer (10 mM Tris-HCl pH 7.4, 10 mM NaCl, 3 mM MgCl2, 0.1% NP40, 0.1% Tween20, 0.01% Digitonin, 1% BSA), and incubated on ice for 3 minutes for permeabilization. After treatment, the cells were washed with washing buffer (10 mM Tris-HCl pH 7.4, 10 mM NaCl, 3 mM MgCl2, 1% BSA), and blocked by incubation on ice for 10 minutes in intracellular staining buffer (BioLegend) containing TruStain FcX (BioLegend). After blocking, the cells were stained by incubation on ice for 45 minutes using an anti-FoxP3 antibody (259D). The stained cells were subjected to sorting of a live FoxP3-positive fraction using FACS Aria Fusion. From the sorted FoxP3-positive fraction, a solution containing sheared chromatin was prepared in the same manner as in Example 10. To the prepared sheared chromatin solution, 10x ChIP dilution buffer (16.7 mM Tris pH 7.5, 167 mM NaCl, 1.2 mM EDTA, 1.1% TritonX-100, 0.01% SDS) in an amount of 1/10 of the solution and cOmplete protease inhibitor cocktail were added, and the solution was further blocked by incubation on ice for at least 1 hour using Fab Fragment Goat Anti-Mouse IgG (Jackson ImmunoResearch). Thereafter, immunoprecipitation, library preparation, DNA sequencing, and data analysis were carried out in the same manner as in Example 10.

As a result, it was confirmed that, in induced Treg cells in which the RBPJ gene was overexpressed, acetylation of histone H3 was suppressed at the FoxP3 gene locus as compared with the negative control (Fig. 11). This result supports, similarly to Example 10, that FoxP3 expression in induced Treg cells is controlled by histone H3 acetylation at the FoxP3 gene locus and in the vicinity thereof by the SMRT-NCoR repressive complex including RBPJ and HDAC3.

## Claims

1. A method for producing regulatory T (Treg) cells, comprising the step of suppressing the activity of RBPJ in conventional T (Tconv) cells.

2. The method according to claim 1, further comprising the step of inducing Treg cells from the T conv cells.

3. The method according to claim 2, wherein the step of inducing Treg cells from the Tconv cells comprises the step of allowing IL-2 and TGF-$\beta$ to act on the Tconv cells.

4. The method according to claim 1, wherein the step of suppressing the activity of RBPJ is the step of suppressing the expression of the RBPJ gene or suppressing the function of the RBPJ protein.

5. The method according to claim 4, wherein the step of suppressing the expression of the RBPJ gene is the step of knocking out the RBPJ gene or knocking down the RBPJ gene.

6. The method according to claim 4, wherein the step of suppressing the function of the RBPJ protein is the step of inhibiting transcriptional activity of the RBPJ protein or inhibiting formation of a complex comprising RBPJ protein, SHARP protein, NCoR1 or NCoR2 protein, GPS2 protein, and HDAC3 protein.

7. The method according to claim 1, wherein the Treg cells are induced Treg (iTreg) cells.

8. The method according to claim 1, wherein the Tconv cells are CD4$^+$ cells, CD8$^+$ cells, CD4$^+$CD25$^-$ cells, or CD4$^+$CD25$^-$ CD45RA$^+$ cells.

9. The method according to claim 1, wherein the Tconv cells are human Tconv cells and the Treg cells are human Treg cells.

10. The method according to claim 1, wherein the method is conducted *in vitro.*

11. The method according to claim 1, wherein when Treg cells produced from Tconv cells in which the activity of RBPJ is suppressed are compared with Treg cells produced from Tconv cells in which the activity of RBPJ is not suppressed, the former Treg cells exhibit any one of the characteristics (i) to (v) below:

    (i) a higher level of FoxP3 expression;
    (ii) a more stable level of FoxP3 expression;
    (iii) enhanced demethylation in the conserved non-coding sequence 2 (CNS2) region of the FoxP3 gene;
    (iv) stronger immunosuppressive activity; and
    (v) more stable immunosuppressive activity.

12. An agent for inducing Treg cells, comprising, as an active ingredient, a compound that suppresses the activity of RBPJ.

13. An agent for inducing Treg cells, comprising, as an active ingredient, Tconv cells in which the activity of RBPJ is suppressed.

14. Treg cells produced by the method of claim 1.

15. An agent for immunosuppression which comprises any one of the following (a) to (c) as an active ingredient:

    (a) Treg cells of claim 14;
    (b) a compound that suppresses the activity of RBPJ, and
    (c) Tconv cells in which the activity of RBPJ is suppressed.

16. The agent according to claim 15, for treating a subject having an autoimmune disease.

17. The agent according to claim 16, wherein the autoimmune disease is selected from the group consisting of Addison's disease, ankylosing spondylitis, autoimmune hepatitis, dermatitis, Goodpasture syndrome, Graves' disease, Guillain-Barré syndrome, Hashimoto's thyroiditis, hemolytic anemia, juvenile arthritis, juvenile myositis, Kawasaki disease, inflammatory bowel disease (such as Crohn's disease and ulcerative colitis), polymyositis, alveolar proteinosis, multiple sclerosis, myasthenia gravis, neuromyelitis optica, psoriasis vulgaris, psoriatic arthritis, rheumatoid arthritis, Sjögren's syndrome, systemic scleroderma, systemic sclerosis, systemic lupus erythematosus, thrombotic thrombocytopenic purpura (TTP), type I diabetes, uveitis, vasculitis, and vitiligo vulgaris.

18. The agent according to claim 15, for treating a subject undergoing tissue transplantation.

19. The agent according to claim 18, which is for suppressing graft rejection and/or graft-versus-host disease (GVHD) in the subject undergoing tissue transplantation.

20. A method for screening an agent for immunosuppression, comprising the step of selecting a compound that suppresses the activity of RBPJ.

21. The method according to claim 20, which comprises the steps of

    (a) measuring the activity of RBPJ in the presence and absence of a test compound; and
    (b) selecting the compound when the activity of RBPJ is suppressed in the presence of the test compound compared with in the absence of the test compound.

22. The method according to claim 21, wherein the measurement is conducted by using cells that express RBPJ.

23. The method according to claim 21, wherein the test compound is selected from the group consisting of: an organic compound, an inorganic compound, a nucleic acid (such as siRNA, shRNA, miRNA, antisense, decoy, aptamer, and ribozyme), a protein (such as an antibody), a peptide (linear peptide, cyclic peptide), a sugar chain, a lipid, and a combination thereof.

Fig. 1

Fig. 2

Fig. 3

Fig. 4

Fig. 5

— PBMC

— PBMC + endogenous T reg cells

— · — PBMC + induced T reg cells (negative control)

— · — PBMC + induced T reg cells (RBPJ KO)

Fig. 6

Fig. 7

Fig. 8

Fig. 9

Fig. 10

<FOXP3

Fig. 11

<FOXP3

# EP 4 782 535 A1

## INTERNATIONAL SEARCH REPORT

| International application No. |
|---|
| **PCT/JP2024/033571** |

### A. CLASSIFICATION OF SUBJECT MATTER

*C12N 5/0783*(2010.01)i; *A61K 35/17*(2025.01)i; *A61K 45/00*(2006.01)i; *A61P 1/04*(2006.01)i; *A61P 1/16*(2006.01)i; *A61P 3/10*(2006.01)i; *A61P 5/14*(2006.01)i; *A61P 7/04*(2006.01)i; *A61P 7/06*(2006.01)i; *A61P 17/00*(2006.01)i; *A61P 17/06*(2006.01)i; *A61P 19/02*(2006.01)i; *A61P 21/00*(2006.01)i; *A61P 29/00*(2006.01)i; *A61P 37/06*(2006.01)i; *C12N 5/10*(2006.01)i; *C12N 15/113*(2010.01)i; *C12N 15/12*(2006.01)i; *C12Q 1/02*(2006.01)i

FI: C12N5/0783 ZNA; A61K35/17; A61K45/00; A61P1/04; A61P1/16; A61P3/10; A61P5/14; A61P7/04; A61P7/06; A61P17/00; A61P17/06; A61P19/02; A61P21/00; A61P29/00 101; A61P37/06; C12N5/10; C12N15/113 Z; C12N15/12; C12Q1/02

According to International Patent Classification (IPC) or to both national classification and IPC

### B. FIELDS SEARCHED

Minimum documentation searched (classification system followed by classification symbols)

C12N5/0783; A61K35/17; A61K45/00; A61P1/04; A61P1/16; A61P3/10; A61P5/14; A61P7/04; A61P7/06; A61P17/00; A61P17/06; A61P19/02; A61P21/00; A61P29/00; A61P37/06; C12N5/10; C12N15/113; C12N15/12; C12Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | DELACHER, M. et al., Rbpj expression in regulatory T cells is critical for restraining TH2 responses, Nature Communications, 2019, 10(1):1621, pp. 1-20, DOI: 10.1038/s41467-019-09276-w<br>page 2, left column | 14 |
| Y | WO 2018/117090 A1 (KYOTO UNIVERSITY) 28 June 2018 (2018-06-28)<br>paragraphs [0002], [0011], examples, claims | 1-23 |
| Y | OU-YANG, H. et al., Notch signaling regulates the FOXP3 promoter through RBP-J- and Hes1-dependent mechanisms, Molecular and Cellular Biology, 2009, vol. 320, pp. 109-114<br>page 113, right column | 1-23 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **28 October 2024** | **12 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

25

**INTERNATIONAL SEARCH REPORT**

| International application No. |
| --- |
| **PCT/JP2024/033571** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2021/209627 A1 (CANCER RESEARCH TECHNOLOGY LIMITED) 21 October 2021 (2021-10-21)<br>        examples, claims | 1-23 |
| A | WO 2007/037544 A1 (KYOTO UNIVERSITY) 05 April 2007 (2007-04-05)<br>        examples, claims | 1-23 |
| A | CN 112274643 A (WEST CHINA HOSPITAL, SICHUAN UNIVERSITY) 29 January 2021 (2021-01-29)<br>        claims, examples | 1-23 |

Form PCT/ISA/210 (second sheet) (July 2022)

## INTERNATIONAL SEARCH REPORT

<table>
<tr><td>International application No.</td></tr>
<tr><td>**PCT/JP2024/033571**</td></tr>
</table>

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    [✓]   forming part of the international application as filed.

     b.    [ ]   furnished subsequent to the international filing date for the purposes of international search (Rule 13*ter*.1(a)),

           [ ]   accompanied by a statement to the effect that the sequence listing does not go beyond the disclosure in the international application as filed.

2.   [ ]   With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT
### Information on patent family members

| International application No. |
| PCT/JP2024/033571 |

| Patent document cited in search report | | | Publication date (day/month/year) | Patent family member(s) | | | Publication date (day/month/year) |
|---|---|---|---|---|---|---|---|
| WO | 2018/117090 | A1 | 28 June 2018 | US | 2020/0095549 | A1 | |
| | | | | paragraphs [0002], [0013], examples, claims | | | |
| WO | 2021/209627 | A1 | 21 October 2021 | JP | 2023-521436 | A | |
| | | | | US | 2023/0158073 | A1 | |
| | | | | EP | 4135725 | A1 | |
| | | | | AU | 2021255917 | A1 | |
| | | | | BR | 112022020819 | A2 | |
| | | | | IL | 297309 | A | |
| | | | | KR | 10-2023-0004643 | A | |
| | | | | CA | 3175622 | A1 | |
| | | | | MX | 2022012924 | A | |
| WO | 2007/037544 | A1 | 05 April 2007 | US | 2009/0246212 | A1 | |
| | | | | examples, claims | | | |
| | | | | EP | 1930415 | A1 | |
| CN | 112274643 | A | 29 January 2021 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016010148 A
- WO 2016010153 A
- WO 2016010154 A
- WO 2016010155 A
- WO 2017159087 A
- WO 2017159088 A
- WO 2017179720 A

**Non-patent literature cited in the description**

- **SCHMIDT A** ; **ERIKSSON M et al.** Comparative analysis of protocols to induce human CD4+FoxP3+ regulatory T cells by combinations of IL-2, TGF-beta, retinoic acid, rapamycin and butyrate.. *PLoS One.*, 2016, vol. 11 (2), e0148474
- **HIPPEN K L** ; **MERKEL S C et al.** Generation and large-scale expansion of human induced regulatory T cells that suppress graft-versus-host disease.. *Am J Transplant.*, 2011, vol. 11 (6), 1148-57
- **HESTER J** ; **SCHIOPU A et al.** Low-dose rapamycin treatment increases the ability of human regulatory T cells to inhibit transplant arteriosclerosis in vivo.. *Am J Transplant.*, 2012, vol. 12 (8), 2008-2016
- **OHKURA et al.** *Immunity.*, 16 November 2012, vol. 37 (5), 785-99
- **YUAN et al.** *Cell Rep.*, 22 January 2019, vol. 26 (4), 845-854
- *Nat Rev Mol Cell Biol.*, April 2015, vol. 16 (4), 258-64